# EUROPEAN PATENT SPECIFICATION

(11) **EP 1 855 585 B1**
(45) Date of publication and mention of the grant of the patent: **07.04.2021**
(21) Application number: 06720920.5
(22) Date of filing: 21.02.2006
(51) Int. Cl.: A61B 5/00

(54) **ICONIC DISPLAY OF MARKERS FOR A METER**
IKON-ANZEIGE VON MARKERN FÜR EIN MESSGERÄT
AFFICHAGE ICONIQUE DE MARQUEURS POUR INSTRUMENT DE MESURE

(30) Priority: 22.02.2005 US 655104 P
(43) Date of publication of application: 21.11.2007
(60) Divisional application: 11179447.5
(73) Proprietor: Ascensia Diabetes Care Holdings AG, 4052 Basel (CH)
(72) Inventor: GUARINO, Julianne, M., Edwardsburg, MI 49112 (US); HUANG, Dijia, Granger, Indiana 46530 (US); BROWN, Daniel, V., Chappaqua, New York 10514 (US); BURNS, Lawrence, J., Elkhart, Indiana 46517 (US)
(74) Representative: Cohausz & Florack
(86) International application number: PCT/US2006/006036
(87) International publication number: WO 2006/091564

(56) References cited:
- EP-A- 1 369 688
- EP-A2- 0 290 683
- WO-A-03/082091
- US-A- 5 899 855
- US-A1- 2004 044 272
- US-A1- 2004 204 635

## Description

### FIELD OF THE INVENTION

The present invention generally relates to an iconic display of markers for use in a device that is adapted to determine at least one analyte concentration in a fluid sample. More particularly, the present invention relates to an iconic display of pre-event and post-event markers for a meter that is adapted to be used to determine at least one analyte concentration in a fluid such as, for example, blood-glucose.

### BACKGROUND OF THE INVENTION

The quantitative determination of analytes in body fluids is of great importance in the diagnoses and maintenance of certain physiological abnormalities. For example, lactate, cholesterol, and bilirubin should be monitored in certain individuals. In particular, determining glucose in body fluids is important to diabetic individuals who must frequently check the glucose level in their body fluids to regulate the glucose intake in their diets. While the remainder of the disclosure herein will be directed towards an iconic display of pre-event and post-event markers for use in glucose meters, it is to be understood that it may be implemented in meters used for determining other analytes.

In one type of blood-glucose testing system, test-sensors are used to test the sample of blood. The results of such tests can be used to determine what, if any, insulin or other medication needs to be administered. Diabetic individuals often test their blood-glucose levels both pre-event and post-event via a blood-glucose meter. Such events may include meals, exercise, illness, stress, symptoms, tracking ketones in urine, or other events that may change their blood-glucose levels. For example, an individual may test his blood-glucose level before eating to assist in determining what amount of sugar is safe to consume during that meal. The individual may also test his blood-glucose after eating to determine whether he consumed too much or too little sugar during his meal. If the results of the test show that his level of glucose is too low or too high, he may need to ingest diabetes pills or insulin doses or use an insulin pump. Similarly, a diabetic individual may test his or her blood-glucose level before and after exercising to ensure that his blood-glucose is at a safe level.

Some existing glucose meters allow an individual to store past glucose readings and other information associated with the reading, including, for example, the date and time. Often, it is important for the individual to store these readings for future reference. Physicians may review this stored information to assist in diagnosing and monitoring the health of their patients.

Today, glucose meters that include meal markers present difficulties for certain users desiring to differentiate between pre-meal and post-meal glucose readings. For example, some meters utilizing iconic meal markers include only one iconic marker denoting a meal without differentiating between pre-meal and post-meal readings. Another type of glucose meter displays pre-meal and post-meal markers via text, thus making it difficult for children or individuals not able to read that particular language to read and understand. Furthermore, document EP 1 369 688 A2 relates to a testing device for testing analyte levels in bodily fluids, wherein the testing device comprises memory for storing data, said data being analyte data related to analyte measurements carried out by the meter and lifestyle data, wherein the testing device comprises initiation means for initiating entry of data related to a specific category of lifestyle data; navigation means for entry and navigation of said data; and transfer means for transferring said data to said memory.

Therefore, it would be desirable to overcome such disadvantages in existing glucose meters.

### SUMMARY OF THE INVENTION

The invention is defined by a meter according to claim 1 and a method according to claim 11. A meter adapted to determine and store an analyte concentration from a fluid sample located on a test sensor is disclosed. The meter includes a display, at least one segment on the display adapted to show a pre-event iconic marker and a post-event iconic marker, at least one button, and a memory device. The display is adapted to display information to a user of the meter. The button allows the user to interact with the meter. The memory device is adapted to store the analyte concentration determination. The user may mark the analyte concentration with the corresponding pre-event or post-event iconic markers by pressing the button.

A method for marking an analyte concentration is also disclosed. The method comprises placing a fluid on a test strip, contacting the test strip with a meter, displaying the analyte concentration of the fluid on a display, selecting a pre-event iconic marker or a post-event iconic marker, and storing the analyte concentration and event iconic marker in the meter. The meter is adapted to determine the analyte concentration of the fluid.

The above summary of the present invention is not intended to represent each embodiment, or every aspect, of the present invention. Additional features and benefits of the present invention are apparent from the detailed description and figures set forth below.

### BRIEF DESCRIPTION OF THE DRAWINGS

FIG. 1a is a test-sensor including a lid according to one embodiment;
FIG. 1b is the test-sensor of FIG. 1a without the lid;
FIG. 2a is a front view of a meter according to one embodiment where all display segments are simultaneously displayed, including pre-meal and post-meal iconic markers in the form of a plate, fork, and knife according to one embodiment.
FIG. 2b is a front view of the meter of FIG. 2a without any meal markers displayed.
FIG. 2c is a front view of the meter of FIG. 2a wherein a pre-meal plate, fork, and knife iconic marker is displayed.
FIG. 2d is a front view of the meter of FIG. 2a wherein a post-meal plate, fork, and knife iconic marker is displayed.
FIG. 3a shows a banana pre-meal iconic marker and a banana peel post-meal iconic marker.
FIG. 3b shows a slice of bread pre-meal iconic marker and a bread crust post-meal iconic marker.
FIG. 3c shows a candy bar pre-meal iconic marker and a candy bar wrapper post-meal iconic marker.
FIG. 3d shows a fish pre-meal iconic marker and a fish bones post-meal iconic marker.
FIG. 3e shows a full glass pre-meal iconic marker and an empty glass post-meal iconic marker.
FIG. 3f shows a whole pizza/pie/cookie pre-meal iconic marker and a slice of pizza/pie/cookie post-meal iconic marker.
FIG. 3g shows a popsicle pre-meal iconic marker and a popsicle stick post-meal iconic marker.
FIG. 3h shows a full spoon pre-meal iconic marker and an empty spoon post-meal iconic marker.
FIG. 3i shows a turkey leg pre-meal iconic marker and a bone post-meal iconic marker.
FIG. 3j shows a slice of watermelon pre-meal iconic marker and a watermelon rind post-meal iconic marker.
FIG. 3k shows a fork and knife placed side-by-side pre-meal iconic marker and a crossed fork and knife post-meal iconic marker.
FIG. 3l shows an apple pre-meal iconic marker and an apple core post-meal iconic marker.
FIG. 4 shows the meter of FIG. 2a including a data port.
FIG. 5a is a front view of a meter according to another embodiment where a fish pre-meal iconic marker is displayed.
FIG. 5b is a front view of the meter of FIG. 5a where a fish bones post-meal iconic marker is displayed.
FIG. 5c shows the meter of FIGS. 5a and 5b where all of the display segments are simultaneously displayed, including the overlaid pre-meal fish and the post-meal fish bones iconic markers.
FIG. 6a is a front view of a meter according to another embodiment where all display segments are simultaneously displayed, including two pairs of pre-meal and post-meal iconic markers.
FIG. 6b is a front view of a meter according to another embodiment where all display segments are simultaneously displayed including two pairs of overlaid pre-meal and post-meal iconic markers.

### DETAILED DESCRIPTION OF THE ILLUSTRATED EMBODIMENTS

The present invention is directed to an iconic display of pre-event and post-event markers for use in a device that is adapted to determine the analyte concentration in a fluid sample. The device contains electrochemical test-sensors that are used to determine concentrations of at least one analyte in a fluid. Analytes that may be determined using the device include glucose, lipid profiles (*e.g.*, cholesterol, triglycerides, LDL and HDL), microalbumin, hemoglobin A₁C, fructose, lactate, or bilirubin. The present invention is not limited, however, to devices for determining these specific analytes and it is contemplated that other analyte concentrations may be determined. The analytes may be in, for example, a whole blood sample, a blood serum sample, a blood plasma sample, or other body fluids like ISF (interstitial fluid) and urine.

According to one embodiment, the test-sensors to be used in the devices are typically provided with a capillary channel that extends from the front or testing end of the sensors to biosensing or reagent material disposed in the sensor. When the testing end of the sensor is placed into fluid (*e.g.*, blood that is accumulated on a person's finger after the finger has been pricked), a portion of the fluid is drawn into the capillary channel by capillary action. The fluid then chemically reacts with the reagent material in the sensor so that an electrical signal indicative of the analyte (*e.g*., glucose) level in the fluid being tested is supplied and subsequently transmitted to an electrical assembly.

Reagent material that may be used to determine the glucose concentration include glucose oxidase. It is contemplated that other reagent material may be used to determine the glucose concentration such as glucose dehydrogenase. It is further contemplated that other reagent material may be used to assist in determining glucose such as, for example, pyrrolo-quinoline quinone glucose dehydrogenase and potassium ferricyanide. The selected reagent may influence items such as the amount of fluid needed and the length of time needed to perform the testing to determine the analyte concentration. If an analyte other than glucose is being tested, different reagent material will likely be used.

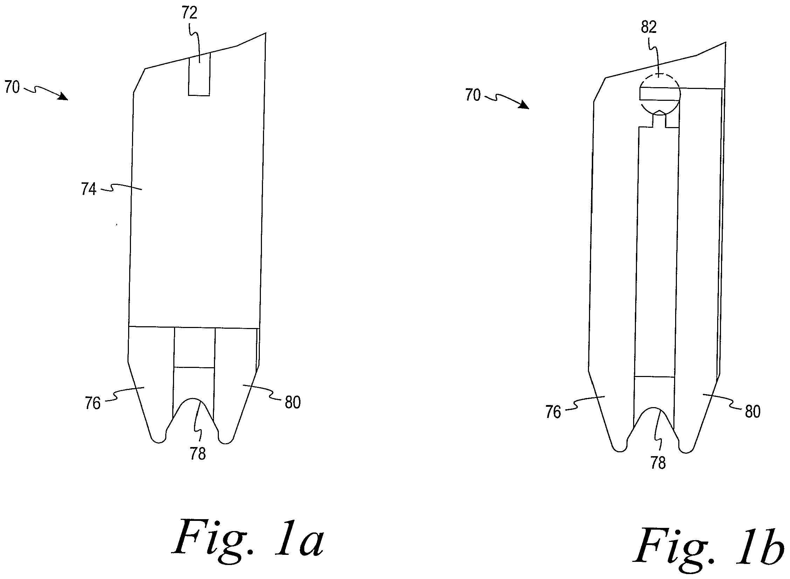

One non-limiting example of a test-sensor is shown in FIGs. 1a, 1b. FIGs. 1a, 1b depict a test-sensor 70 that includes a capillary channel 72, a lid 74, and a plurality of electrodes 76, 78, and 80. The plurality of electrodes includes a counter electrode 76, a detection electrode 78, and a working (measuring) electrode 80. As shown in FIG. 1b, the test-sensor 70 includes a fluid-receiving area 82 that contains reagent. The operation of fluid-receiving area with reagent and the electrodes on the test-sensors is known to those skilled in the art and will therefore not be described in further detail. Examples of electrochemical test-sensors, including their operation, may be found at, for example, U.S. Patent Application published as 2001/0042683 and EP 1152239. It is contemplated that other electrochemical test-sensors may be employed.

The test-sensors are not limited to electrochemical test-sensors. For example, it is contemplated that optical test sensors may be used in the present invention.

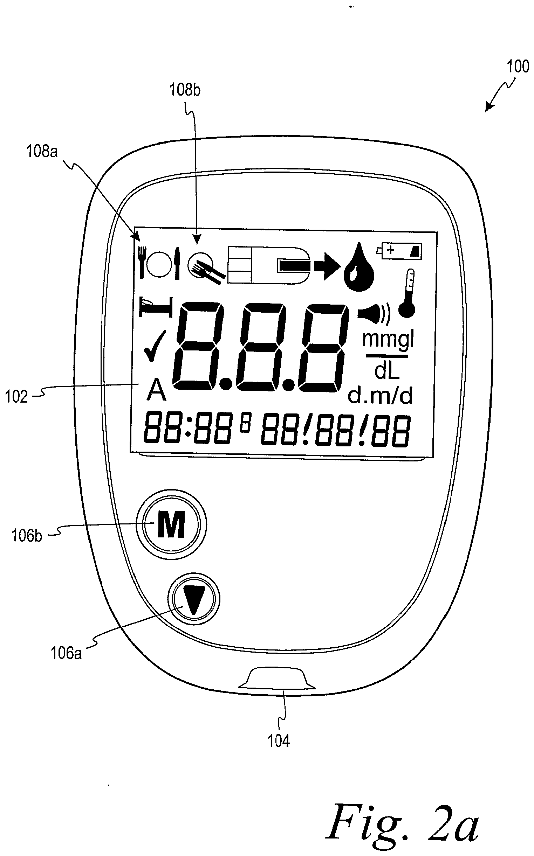

Referring to FIGS. 2a-d, an iconic display of meal markers is shown on a glucose meter 100 according to one embodiment. As shown in FIG. 2a, the meter 100 includes a display 102, a test-sensor dispensing port 104, and a plurality of buttons 106. FIG. 2a shows the meter 100 with all of the display segments shown. The display 102 includes iconic markers 108 so that
the user is able to mark whether a particular glucose reading was taken pre-event or post-event. In this embodiment, plate iconic markers 108 are used to mark whether the glucose reading was taken pre-meal or post-meal. A pre-meal iconic marker 108a is a plate with a knife and fork on either side of it, which is typically the way a plate, knife, and fork are arranged before a meal. A post-meal iconic marker 108b is a plate with a knife and fork positioned side-by-side on the plate at an angle, a position typically used to denote that one is finished with his meal.

After a user places a fluid (*e.g.*, his blood) on a test-sensor, the glucose level is determined by the meter 100, which displays the glucose reading on the display 102. The user may then press buttons 106a-b to mark the reading accordingly based on whether the reading was taken before or after eating.

It is contemplated that the user may mark the reading by other means than the previously described buttons 106a and 106b. Such other means include but are not limited to a touch screen, a single button, a dial, a toggle switch, preset mealtimes in the meter, and auto mark.

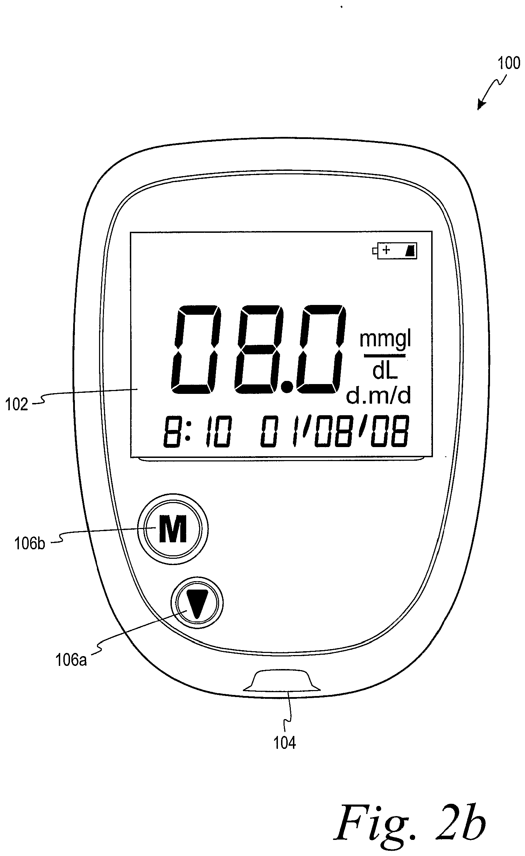

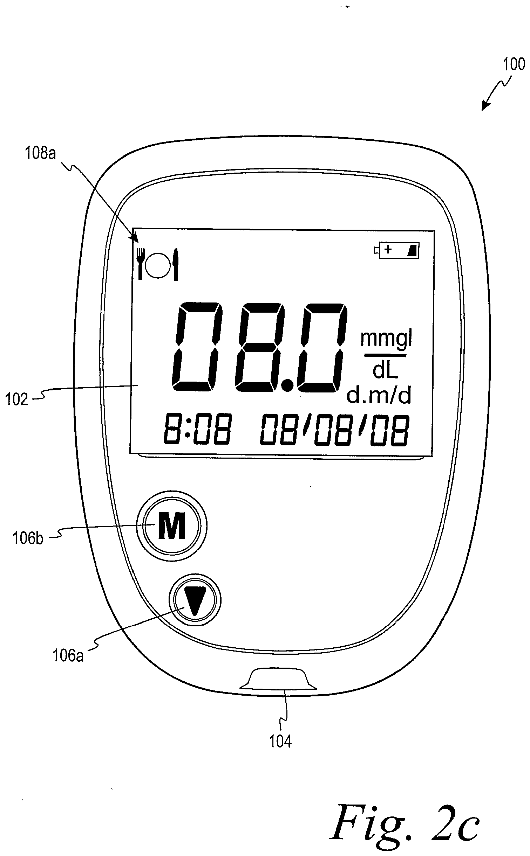

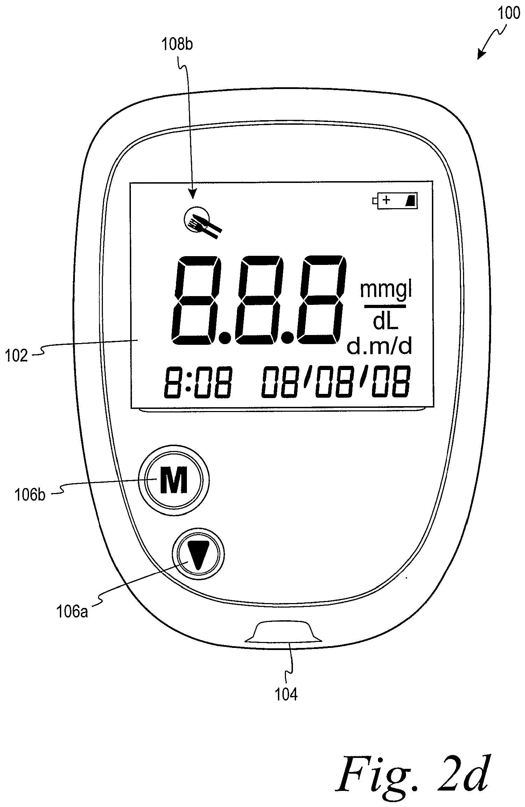

As shown in FIG. 2b, initially, after the glucose reading is taken, no meal marker is displayed according to one embodiment. When the user first presses the button 106a, the pre-meal iconic marker 108a appears on the display 102, as show in FIG. 2c. When the user presses the button 106a again, the pre-meal marker 108a disappears, and the post-meal marker 108b appears on the display 102, as show in FIG. 2d. When the button 106a is pressed a third time, the post-meal marker 108b disappears so that neither marker 108a, 108b is displayed on the display 102, and the display appears the way it initially looked after the reading was taken, as shown in FIG. 2b. Pressing the button 106a again repeats the cycle. When the proper marker is displayed, the user may select it by, for example, pressing another button 106b or letting the meter time out. The user may also choose not to mark the reading with either iconic marker 108a, 108b by pressing the button 106b while neither marker is displayed or letting the meter time out. The glucose reading is then stored in the meter's memory device with the selected iconic markers. The user may then go back at a later time to review and compare glucose readings.

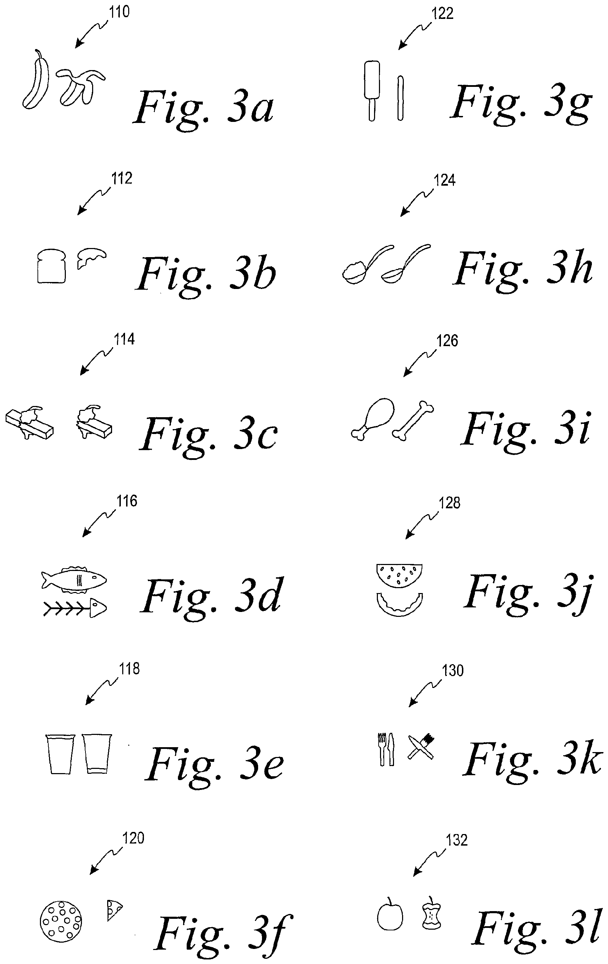

Other iconic markers may also be used to represent pre-meal and post-meal readings, including, but not limited to, the iconic markers shown in FIG. 3a-3l. Pre-meal and post-meal iconic marker pairs may include a banana and a banana peel 110 (FIG. 3a), a slice of bread and bread crust 112 (FIG. 3b), a candy bar and a wrapper 114 (FIG. 3c), a fish and fish bones 116 (FIG. 3d), a full glass and an empty glass 118 (FIG. 3e), a whole pizza/pie/cookie and a slice of pizza/pie/cookie 120 (FIG. 3f), a popsicle and a popsicle stick 122 (FIG. 3g), a full spoon and an empty spoon 124 (FIG. 3h), a turkey leg and bone 126 (FIG. 3i), a slice of watermelon and rind 128 (FIG. 3j), a fork and knife placed side-by-side and a crossed fork and knife 130 (FIG. 3k), and an apple and an apple core 132 (FIG. 3l).

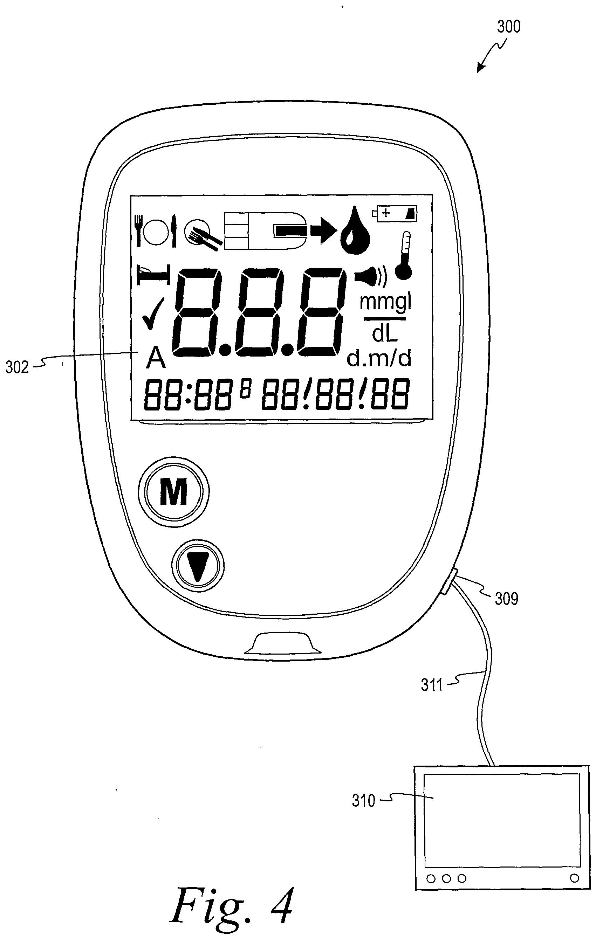

In the embodiment of FIG. 4, a meter 300 may include a data port 309, which is connected to, for example, a personal computer 310 via a cable or cord 311. The data port 309 allows the meter 300 to communicate with the personal computer 310 so that the stored glucose readings and corresponding information could be transferred to the computer 310.

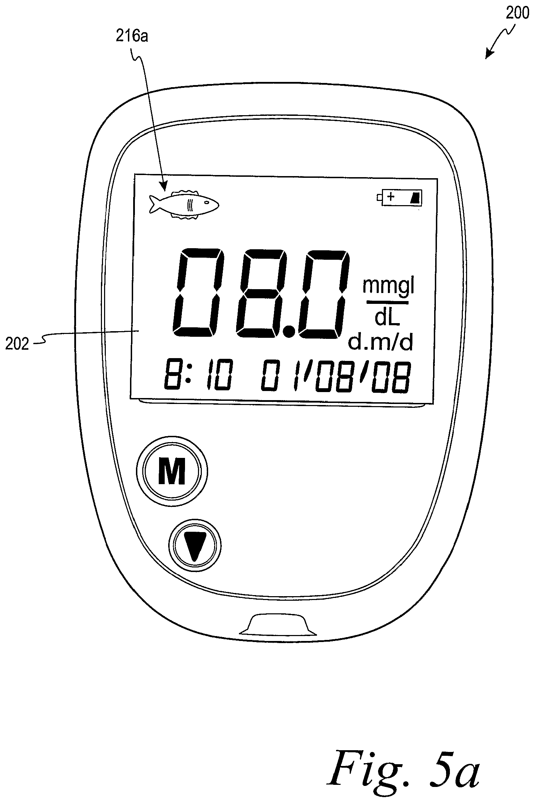

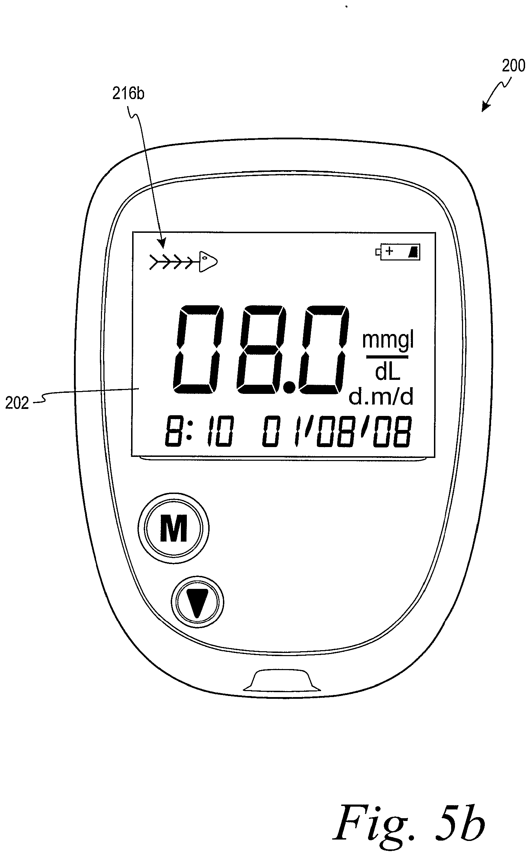

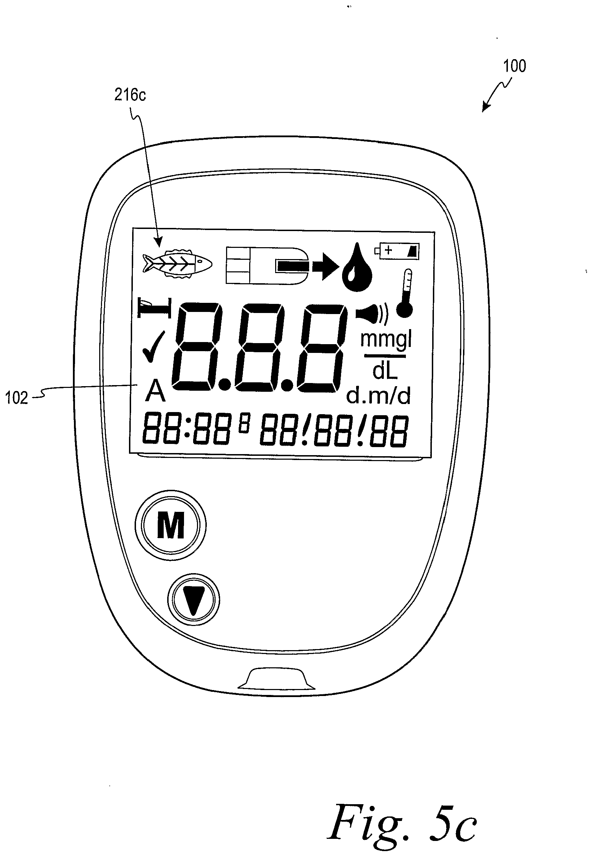

Referring now to the embodiment of FIG. 5a-c, a meter display may be designed such that the pre-meal and post-meal iconic markers are overlaid. A meter 200 of FIG. 5a includes a fish pre-meal iconic marker 216a. FIG. 5b shows corresponding fish bones post-meal iconic marker 216b. FIG. 5c shows the meter display 202 if both the pre-meal and post-meal iconic markers were displayed at once. The pre-meal fish iconic marker 216a and the post-meal fish iconic marker 216b are overlaid to produce the overlaid iconic marker 216c. Overlaying the iconic markers is beneficial because it requires less space on the meter display to be taken up by the pre-meal and post-meal iconic markers and, thus, potentially enables the other features of the display to be more prominent or spaced further apart.

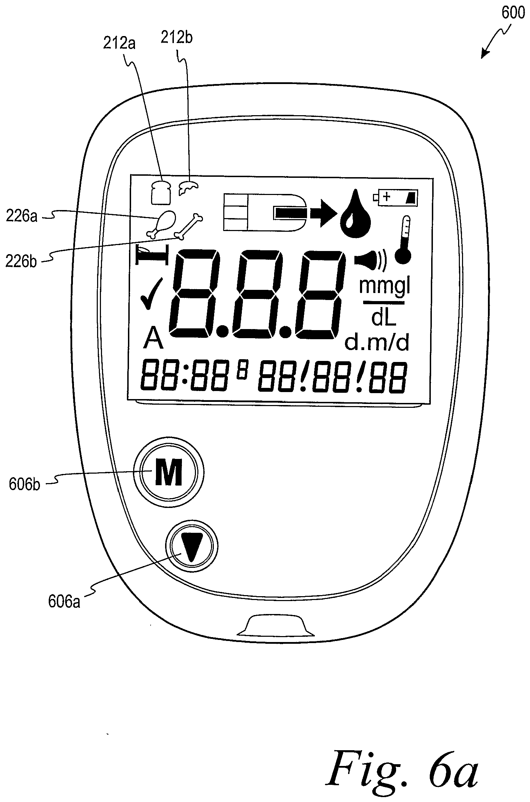

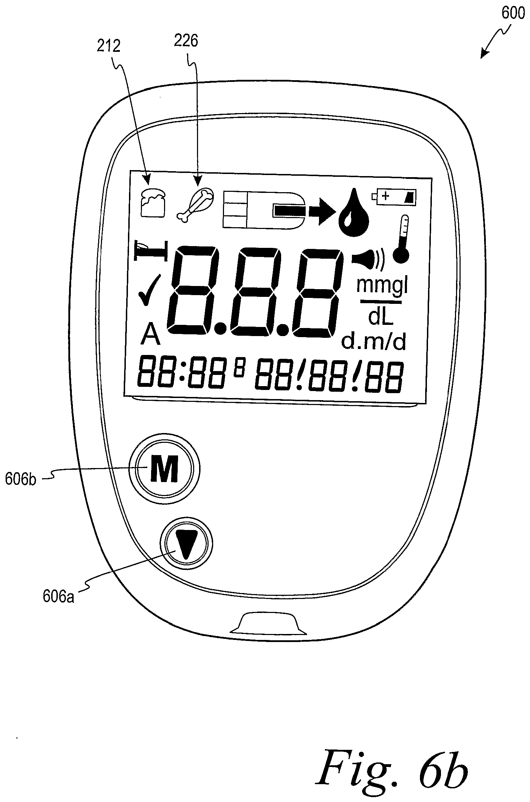

In an alternative embodiment shown in FIG. 6a, the meter 600 comes equipped with several different pre-meal and post-meal iconic markers for the user to choose from. For example, the user may choose from several of the pairs of iconic markers shown in FIG. 3a-3l. The user may press a button 606a on the meter to scroll through the available iconic markers to mark the reading. To select one, the user may press another button 606b. For example, the user can choose the bread iconic markers 212 or the turkey iconic markers 226 to denote pre-meal or post-meal readings. This embodiment may be less desirable since it requires a more complex display to be used in order to accommodate the different iconic marker selections. Alternatively, the pairs of iconic markers that the user may select from may be overlaid, as shown in the embodiment of FIG. 6b.

### Alternative Embodiment A

A meter adapted to determine and store an analyte concentration from a fluid sample located on a test sensor, the meter comprising:
a display adapted to display information to a user of the meter;
at least one segment on the display adapted to show a pre-event iconic marker and a post-event iconic marker;
at least one button for allowing the user to interact with the meter; and
a memory device adapted to store the analyte concentration determination,
wherein the user may mark the analyte concentration with the corresponding pre-event or post-event iconic markers by pressing the at least one button.

### Alternative Embodiment B

The meter of embodiment A further comprising a test-sensor dispensing port.

### Alternative Embodiment C

The meter of embodiment A further comprising a data port adapted to transfer information to a personal computer.

### Alternative Embodiment D

The meter of embodiment A wherein the iconic markers mark pre-meal and post-meal analyte readings.

### Alternative Embodiment E

The meter of embodiment A wherein the iconic markers mark pre-exercise and post-exercise analyte readings.

### Alternative Embodiment F

The meter of embodiment A wherein the analyte is glucose and the fluid sample is whole blood.

### Alternative Embodiment G

The meter of embodiment A wherein the display includes two segments adapted to display a respective one of the pre-event and post-event iconic markers.

### Alternative Embodiment H

The meter of embodiment A wherein the display includes one segment adapted to display the pre-event and post-event iconic markers, such that the pre-event and post-event iconic markers are overlaid.

### Alternative Embodiment I

The meter of embodiment A wherein the display is adapted to display several different pairs of pre-event and post-event iconic markers.

### Alternative Embodiment J

The meter of embodiment I wherein the pair of pre-event and post-event iconic markers is adapted to be user-selectable.

### Alternative Process K

A method of marking an analyte concentration, the method comprising the acts of:
placing a fluid on a test strip;
contacting the test strip with a meter, the meter being adapted to determine the analyte concentration of the fluid;
displaying the analyte concentration of the fluid on a display;
selecting a pre-event iconic marker or a post-event iconic marker; and
storing the analyte concentration and event iconic marker in the meter.

### Alternative Process L

The method of process K wherein selecting the pre-event iconic marker or post-event iconic marker includes pressing a first button adapted to alternate between displaying a respective one of a pre-event iconic marker, a post-event iconic marker, and a neither event iconic marker each time the first button is pressed and pressing a second button to select the appropriate event iconic marker.

While the invention is susceptible to various modifications and alternative forms, specific embodiments and methods thereof have been shown by way of example in the drawings and are described in detail herein.

## Claims

1. A meter adapted to determine and store an analyte concentration from a fluid sample located on a test sensor, the meter comprising:
a display adapted to display information to a user of the meter;
at least one segment on the display displaying a pre-event iconic marker and a post-event iconic marker, the pre-event iconic marker and the post-event iconic marker being paired non-language based graphical images;
at least one marking means for allowing the user to interact with the meter, wherein after the analyte concentration is taken, no pre-event iconic marker is displayed, wherein a pre-event iconic marker appears on the display upon pressing a button, wherein the pre-event iconic marker disappears and the post-event iconic marker appears on the display upon pressing the button again, wherein when the proper iconic marker is displayed, it is selectable by the user; and
a memory device adapted to store the analyte concentration with the corresponding pre-event or post-event iconic marker.

2. The meter of claim 1, further comprising a test-sensor dispensing port.

3. The meter of claim 1, further comprising a data port adapted to transfer information to a personal computer.

4. The meter of claim 1, wherein the iconic markers mark pre-meal and post-meal analyte readings.

5. The meter of claim 1, wherein the iconic markers mark pre-exercise and post-exercise analyte readings.

6. The meter of claim 1, wherein the analyte is glucose and the fluid sample is whole blood.

7. The meter of claim 1, wherein the display includes two segments adapted to display a respective one of the pre-event and post-event iconic markers.

8. The meter of claim 1, wherein the display includes one segment adapted to display the pre-event and post-event iconic markers, such that the pre-event and post-event iconic markers are overlaid.

9. The meter of claim 1, wherein the display is adapted to display several different pairs of pre-event and post-event iconic markers.

10. The meter of claim 9, wherein the pair of pre-event and post-event iconic markers is adapted to be user-selectable.

11. A method of marking an analyte concentration, the method comprising the acts of:
placing a fluid on a test strip;
contacting the test strip with a meter, the meter being adapted to determine the analyte concentration of the fluid;
displaying the analyte concentration of the fluid on a display;
wherein after the analyte concentration is taken, no pre-event iconic marker is displayed, wherein a pre-event iconic marker appears on the display upon pressing a button, wherein the pre-event iconic marker disappears and the post-event iconic marker appears on the display upon pressing the button again,
wherein when the proper iconic marker is displayed, it is selected by the user, the pre-event iconic marker and the post-event iconic marker being paired non-language based graphical images;
selecting the pre-event iconic marker or the post-event iconic marker; and
storing the analyte concentration and the selected.iconic marker in the meter.

12. The method of claim 11, wherein the marking means includes a first button and a second button, the selecting the pre-event iconic marker or post-event iconic marker including pressing the first button adapted to alternate between displaying a respective one of a pre-event iconic marker, a post-event iconic marker, and a neither event iconic marker each time the first button is pressed and pressing second button to select the appropriate event iconic marker.

## Patentansprüche

1. Messgerät, das zum Bestimmen und zum Speichern einer Analytkonzentration aus einer Fluidprobe eingerichtet ist, die sich auf einem Testsensor befindet, umfassend:
eine Anzeige eingerichtet zum Anzeigen von Informationen einem Benutzer des Messgeräts;
mindestens ein Segment auf der Anzeige, das eine ikonische Vorereignis Markierung und eine ikonische Nachereignis Markierung anzeigt, wobei die ikonische Vorereignis Markierung und die ikonische Nachereignis Markierung gepaarte nicht sprachbasierte grafische Bilder sind;
mindestens eine Markierungseinrichtung zum Ermöglichen eines Interagierens eines Benutzers mit dem Messgerät zu interagieren,
wobei nach der Messung der Analytkonzentration keine ikonische Vorereignis Markierung angezeigt wird, wobei eine ikonische Vorereignis Markierung auf der Anzeige erscheint bei einem Drücken einer Taste, wobei die ikonische Vorereignis Markierung verschwindet und die ikonische Nachereignis Markierung bei einem erneuten Drücken der Taste erscheint, wobei, wenn die richtige ikonische Markierung angezeigt wird, diese von dem Benutzer auswählbar ist; und
eine Speichervorrichtung eingerichtet zum Speichern der Analytkonzentration mit der entsprechenden ikonischen Vorereignis oder Nachereignis Markierung.

2. Messgerät nach Anspruch 1, ferner umfassend eine Testsensor Ausgabeöffnung.

3. Messgerät nach Anspruch 1, ferner umfassend einen Datenanschluss, eingerichtet zum Übertragen von Informationen an einen Personalcomputer.

4. Messgerät nach Anspruch 1, wobei die ikonischen Markierungen Vormahlzeit und Nachmahlzeit Messwerte markieren.

5. Messgerät nach Anspruch 1, wobei die ikonischen Markierungen Vortrainings und Nachtrainings Messwerte markieren.

6. Messgerät nach Anspruch 1, wobei der Analyt Glukose ist und die Fluidprobe Vollblut ist.

7. Messgerät nach Anspruch 1, wobei die Anzeige zwei Segmente umfasst eingerichtet zum Anzeigen einer entsprechenden Markierung der ikonischen Vorereignis und Nachereignis Markierungen.

8. Messgerät nach Anspruch 1, wobei die Anzeige ein Segment umfasst, eingerichtet zum Anzeigen der ikonischen Vorereignis und Nachereignis Markierungen derart, dass die ikonischen Vorereignis und Nachereignis Markierungen überlagert sind.

9. Messgerät nach Anspruch 1, wobei die Anzeige eingerichtet ist zum Anzeigen mehrerer verschiedener Paare von ikonischen Vorereignis und Nachereignis Markierungen.

10. Messgerät nach Anspruch 9, wobei das Paar der ikonischen Vorereignis und Nachereignis Markierungen eingerichtet ist, um durch einen Benutzer auswählbar zu sein.

11. Verfahren zum Markieren einer Analytkonzentration, wobei das Verfahren die folgenden Schritte umfasst:
Auftragen eines Fluids auf einen Teststreifen;
Kontaktieren des Teststreifens mit einem Messgerät, wobei das Messgerät zum Bestimmen der Analytkonzentration des Fluids eingerichtet ist;
Anzeigen der Analytkonzentration des Fluids auf einer Anzeige;
wobei nach der Messung der Analytkonzentration keine ikonische Vorereignis Markierung angezeigt wird, wobei eine ikonische Vorereignis Markierung bei einem Drücken einer Taste auf der Anzeige erscheint, wobei die ikonische Vorereignis Markierung verschwindet und die ikonische Nachereignis Markierung bei einem erneuten Drücken der Taste auf der Anzeige erscheint,
wobei die ikonische Vorereignis Markierung und die ikonische Nachereignis Markierung, wenn die richtige ikonische Markierung angezeigt wird, von dem Benutzer ausgewählt wird, wobei die ikonische Vorereignis Markierung und die ikonische Nachereignis Markierung gepaarte nicht sprachbasierte grafische Bilder sind;
Auswählen der ikonischen Vorereignis Markierung oder der ikonischen Nachereignis Markierung; und
Speichern der Analytkonzentration und der gewählten ikonischen Markierung in dem Messgerät.

12. Verfahren nach Anspruch 11, wobei das Markierungsmittel eine erste Taste und eine zweite Taste umfasst, wobei das Auswählen der ikonischen Vorereignis Markierung oder der ikonischen Nachereignis Markierung ein Drücken der ersten Taste umfasst, die eingerichtet ist, dass sie jedes Mal, wenn die erste Taste gedrückt wird, zwischen der Anzeige einer entsprechenden ikonischen Vorereignis Markierung, einer ikonischen Nachereignis Markierung und einer ikonischen Markierung für keines der Ereignisse wechselt, und ein Drücken der zweiten Taste umfasst, um die entsprechende ikonische Markierung für das Ereignis auszuwählen.

## Revendications

1. Appareil de mesure adapté pour déterminer et stocker une concentration en un matériau à analyser à partir d'un échantillon de fluide situé sur un capteur de test, l'appareil de mesure comprenant :
- un moyen d'affichage adapté pour afficher des informations à un utilisateur de l'appareil de mesure ;
- au moins un segment sur le moyen d'affichage affichant un marqueur iconique pré-événement et un marqueur iconique post- événement, le marqueur iconique pré-événement et le marqueur iconique post-événement étant appariés à des images graphiques non linguistiques;
- au moins un moyen de marquage permettant à l'utilisateur d'interagir avec l'appareil de mesure,
- où, après que la concentration du matériau à analyser est prise, aucun marqueur iconique pré-événement n'est affiché,
- où un marqueur iconique pré-événement apparaît sur le moyen d'affichage en appuyant sur un bouton,
- où le marqueur iconique pré-événement disparaît et le marqueur iconique post-événement apparaît sur le moyen d'affichage en appuyant à nouveau sur le bouton,
- où, lorsque le marqueur iconique approprié est affiché, il est sélectionnable par l'utilisateur ;
et
- un dispositif de mémoire adapté pour stocker la concentration en matériau à analyser avec le marqueur iconique pré-évènement ou post-évènement correspondant.

2. Appareil de mesure la revendication 1, comprenant en outre un port de distribution pour le capteur de test.

3. Appareil de mesure selon la revendication 1, comprenant en outre un port de données adapté pour transférer des informations à un ordinateur personnel.

4. Appareil de mesure de la revendication 1, où les marqueurs iconiques marquent des relevés de matériau à analyser pré-repas et post-repas.

5. Appareil de mesure de la revendication 1, où les marqueurs iconiques marquent des relevés de matériau à analyser pré-effort et post-effort.

6. Appareil de mesure selon la revendication 1, où le matériau à analyser est du glucose et l'échantillon de fluide est du sang entier.

7. Appareil de mesure selon la revendication 1, où le moyen d'affichage comprend deux segments adaptés pour afficher respectivement un des marqueurs pré-effort et post-effort.

8. Appareil de mesure selon la revendication 1, où le moyen d'affichage comprend un segment adapté pour afficher les marqueurs iconiques pré-évènement et post-évènement, de sorte que les marqueurs iconiques pré-évènement et post-évènement sont superposés.

9. Appareil de mesure selon la revendication 1, où le moyen d'affichage est adapté pour afficher plusieurs paires différentes de marqueurs iconiques pré-événement et post-événement.

10. Appareil de mesure selon la revendication 9, où la paire de marqueurs iconiques pré-événement et post-événement est adaptée pour être sélectionnable par l'utilisateur.

11. Méthode de marquage d'une concentration en matériau à analyser, la méthode comprenant les actes de:
- placer un fluide sur une bandelette de test ;
- mettre en contact la bandelette de test avec un appareil de mesure, l'appareil de mesure étant adapté pour déterminer la concentration en matériau à analyser du fluide ;
- afficher la concentration du matériau à analyser du fluide sur un moyen d'affichage;
- où, après que la concentration du matériau à analyser est prise, aucun marqueur iconique pré-événement n'est affiché,
- où un marqueur iconique pré-événement apparaît sur le moyen d'affichage en appuyant sur un bouton,
- où le marqueur iconique pré-événement disparaît et le marqueur iconique post-événement apparaît sur le moyen d'affichage en appuyant à nouveau sur le bouton,
- où, lorsque le marqueur iconique approprié est affiché, il est sélectionné par l'utilisateur, le marqueur iconique pré-événement et le marqueur iconique post-événement étant appariés à des images graphiques non linguistiques;
- sélectionner le marqueur iconique pré-événement ou le marqueur iconique post-événement ; et
- stocker la concentration du matériau à analyser et le marqueur iconique sélectionné dans l'appareil de mesure.

12. Méthode selon la revendication 11, dans laquelle le moyen de marquage comprend un premier bouton et un second bouton, la sélection du marqueur iconique pré-événement ou du marqueur iconique post-événement comprenant la pression du premier bouton adapté pour alterner entre afficher l'un des marqueurs iconiques pré-événement, post-événement et non-événement à chaque fois que le premier bouton est pressé et la pression du second bouton pour sélectionner le marqueur iconique approprié.
